# FASCICULE DE BREVET EUROPEEN

(11) **EP 0 645 626 B1**
(45) Date de publication et mention de la délivrance du brevet: **24.05.2000**
(21) Numéro de dépôt: 94114427.1
(22) Date de dépôt: 14.09.1994
(51) Int. Cl.: G01N 33/487, G01N 27/28, C12Q 1/00

(54) **Dispositif de mesure pour capteurs amovibles**
Messvorrichtung für entfernbare Sensoren
Measuring device for detachable sensors

(30) Priorité: 21.09.1993 FR 9311318
(43) Date de publication de la demande: 29.03.1995
(73) Titulaire: ASULAB S.A., CH-2501 Bienne (CH)
(72) Inventeur: Desarzens, Pierre, CH-2503 Bienne (CH); Dinger, Rudolf, CH-2024 St-Aubin (CH)
(74) Mandataire: Patry, Didier Marcel Pierre

(56) Documents cités:
- EP-A- 0 121 385
- EP-A- 0 429 076
- EP-A- 0 523 463
- WO-A-90/10861
- WO-A-91/00998
- WO-A-93/04371

## Description

La présente invention concerne un dispositif de mesure destiné à être associé à un capteur amovible. Par exemple, un tel dispositif de mesure sert à effectuer des mesures du tue électrochimique, notamment pour mesurer le taux de glucose dans le sang. Le fonctionnement électrochimique pour une telle mesure est décrit par exemple dans la demande de brevet WO 92/14 836.

Plus particulièrement, la présente invention concerne la connexion électrique entre un tel dispositif de mesure et un capteur amovible associé à ce dispositif de mesure.

Sur la figure 1 est représentée de manière schématique une vue générale d'un dispositif de mesure, désigné par la référence générale 2, et d'un capteur amovible désigné par la référence 4.

Le capteur amovible 4 comporte une zone active 6 et deux plages de contact 8 et 10 reliées électriquement à la zone active 6 à l'aide de deux conducteurs non représentés.

Sur la figure 2 est représentée schématiquement une connexion électrique classique entre un dispositif de mesure (partiellement représenté) et un capteur amovible du type représenté à la figure 1.

La connexion électrique est réalisée par deux lames métalliques 12 et 14 associés respectivement aux deux plages de contact 8 et 10, ces dernières étant reliées électriquement en série avec la zone active 6 du capteur amovible 4.

Un tel dispositif correspond par exemple à celui qui est décrit dans le document EP 0 523 463.

Pour permettre au capteur amovible 4 d'être introduit dans le dispositif de mesure ou d'être retiré de ce dernier, la connexion électrique est assurée uniquement par pression des lames métalliques 12 et 14 sur les plages de contact respectives 8 et 10, ces lames 12 et 14 présentant une certaine élasticité.

L'agencement de la connexion électrique décrit ci-avant est peu fiable. En effet, il se peut aisément qu'une des lames métalliques subisse une déformation suite à l'introduction répétée de capteurs amovibles ou que la pression exercée par cette lame métallique soit insuffisante pour établir une connexion électrique correcte. De plus, il se peut que le capteur amovible 4 soit défectueux et que les plages de contact présentent par exemple une résistance électrique hors normes.

Dans le document WO 93/04371, la présence de trois plages de contacts n'augmente pas pour autant la fiabilité du capteur étant donné que chacune d'elles est reliée à un pôle électrique différent de l'appareil de mesure.

Les différents problèmes cités ci-avant sont spécialement néfastes pour une mesure à caractère médical.

Le but de la présente invention est de pallier le manque de sécurité de la connexion électrique entre un dispositif de mesure et un capteur amovible destiné à être associé à ce dispositif de mesure.

A cet effet, la présente invention concerne un dispositif de mesure destiné à être associé à un capteur amovible présentant au moins deux plages de contact électrique, ce dispositif de mesure comprenant des moyens de mesure et des moyens de connexion comportant au moins deux premiers moyens de contact. Ces deux premiers moyens de contact servent respectivement a établir une connexion électrique entre les deux plages de contact dudit capteur amovible et ces moyens de mesure. Ce dispositif de mesure est caractérisé en ce que lesdits moyens de connexion comportent en outre au moins deux deuxièmes moyens de contact, ces deux deuxièmes moyens de contact servant respectivement à relier électriquement les deux plages de contact dudit capteur amovible à des moyens de contrôle permettant de contrôler ladite connexion électrique entre chacune des deux premières plages de contact et lesdits moyens de mesure.

Le doublement des moyens de contact selon l'invention entre le dispositif de mesure et le capteur amovible auquel il est associé permet, selon divers modes de réalisation, d'établir au moins un circuit électrique ne comportant pas la zone active du capteur amovible et permettant de contrôler si la connexion électrique entre les premiers moyens de contact et les plages de contact respectives du capteur amovible est correctement établie, ceci pour autant que la connexion électrique entre les deuxièmes moyens de contact et les plages de contact électriques respectives de ce capteur amovible soit également correctement établie.

De ce fait, il est possible, avant d'effectuer une mesure à l'aide du dispositif de mesure et du capteur amovible qui lui est associé, de s'assurer que l'établissement d'une connexion électrique entre chacun des premiers ou deuxièmes moyens de contact intervenant dans la mesure et le capteur amovible est correctement établie.

Selon une autre caractéristique de l'invention, il est également possible de contrôler si le capteur utilisé pour la mesure est défectueux.

D'autres avantages et caractéristiques de l'invention seront mieux compris à l'aide de la description suivante faite en référence aux dessins annexés, donnés à titre d'exemples nullement limitatifs, dans lesquels :

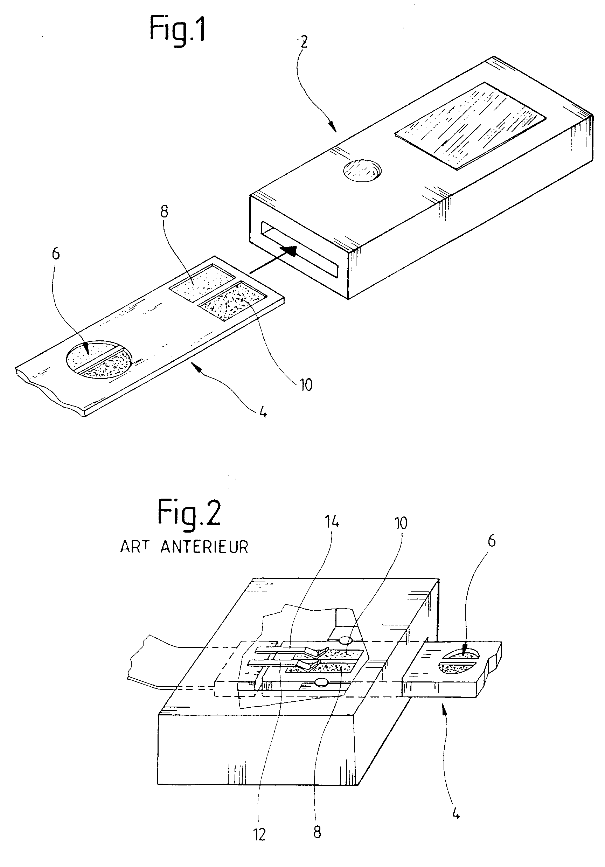

- la figure 1, déjà décrite, est une vue générale en perspective d'un dispositif de mesure et d'un capteur amovible qui lui est associé;
- la figure 2, déjà décrite, est une vue partielle d'un dispositif de mesure de l'art antérieur montrant schématiquement la connexion électrique entre le dispositif de mesure et un capteur amovible qui lui est associé;

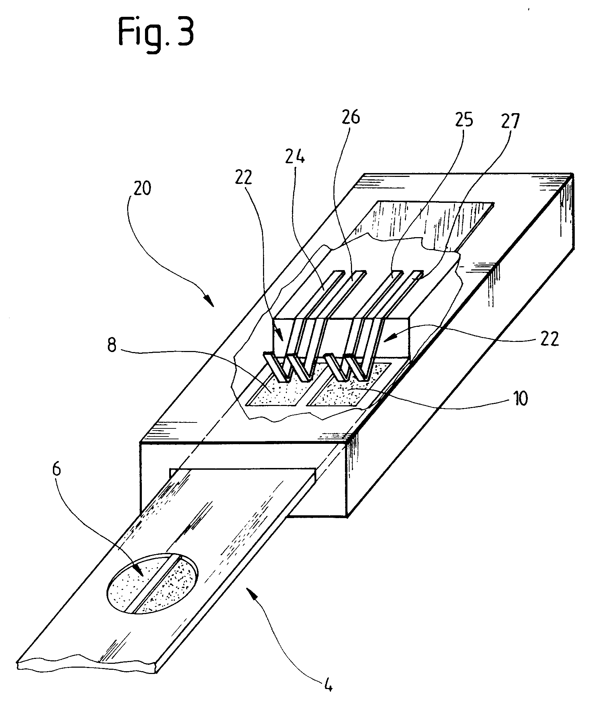

- la figure 3 montre schématiquement en perspective un dispositif de mesure selon l'invention associé à un capteur amovible;

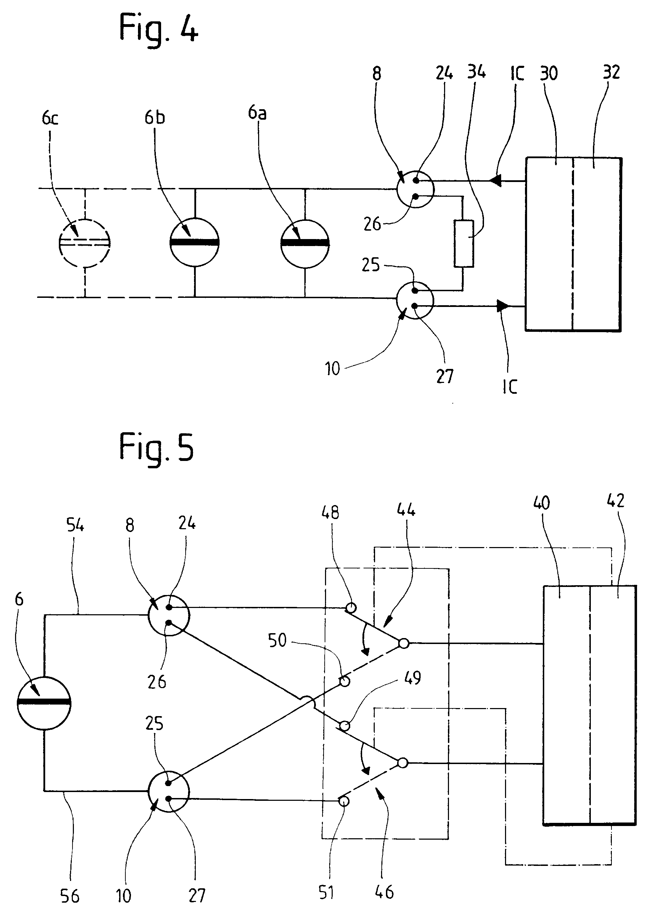

- la figure 4 représente schématiquement un premier mode de réalisation du circuit électrique du dispositif de mesure selon l'invention;
- la figure 5 représente schématiquement un deuxième mode de réalisation du circuit électrique du dispositif de mesure selon l'invention.

Sur la figure 3, le dispositif de mesure selon l'invention, désigné par la référence générale 20, est associé à un capteur amovible 4 comportant au moins une zone active 6 et deux plages de contact électrique 8 et 10. Ces plages de contact électrique 8 et 10 sont isolées électriquement l'une de l'autre et sont reliées électriquement à la zone active 6 au moyen de deux conducteurs respectifs (non représentés) intégrés dans le capteur amovible 4.

Le dispositif de mesure 20 comprend des moyens de connexion 22 formés par quatre contacts électriques 24, 25, 26 et 27 constitués respectivement par quatre lames ou fils métalliques. Ces quatre lames métalliques 24 à 27, servant de moyens de contact électrique entre le dispositif de mesure 20 et le capteur amovible 4, sont recourbés et présentent une certaine élasticité permettant à ces quatre lames métalliques d'être en appui sur les plages de contact électrique 8 et 10 du capteur amovible 4, c'est-à-dire d'exercer une certaine pression de contact sur ces plages de contact électrique 8 et 10.

Ainsi, chacune des deux plages de contact électrique 8 et 10 est associée avec deux contacts électriques 24 et 26, 25 et 27 indépendants l'un de l'autre. Ce doublement des contacts électriques par plage de contact électrique permet de contrôler la connexion électrique entre le capteur amovible 4 et le dispositif de mesure 20 au moyen d'un circuit électrique ou électronique agencé dans ce dispositif de mesure. Ci-après seront décrits deux modes de réalisation de ce circuit électrique du dispositif de mesure 20 selon l'invention.

Sur la figure 4 est représenté un premier mode de réalisation du circuit électrique d'un dispositif de mesure selon l'invention.

Sur cette figure 4 sont représentés schématiquement les deux plages de contact électrique 8 et 10 d'un capteur amovible et les quatre contacts électriques 24, 25, 26 et 27 d'un dispositif de mesure selon l'invention qui leur sont associés.

On notera ici que le capteur amovible est un capteur multizone comprenant une pluralité de zones actives 6a, 6b, 6c reliées électriquement en parallèle aux deux plages de contact électrique 8 et 10, le nombre de ces zones actives étant quelconque. Le circuit électrique du dispositif de mesure selon l'invention comprend des moyens de mesure 30 et des moyens de contrôle comprenant une électronique de contrôle 32 et une résistance de contrôle 34. On notera que les moyens de mesure 30 et l'électronique de contrôle 32 peuvent être formés par une seule et même unité électronique.

Les contacts électriques 24 et 27 associés respectivement aux plages de contact 8 et 10 sont reliés aux moyens de mesure 30. Par contre, les contacts électriques 25 et 26 associés respectivement aux plages de contact électrique 8 et 10 sont reliés à la résistance de contrôle 34. Dans ce mode de réalisation, la valeur de la résistance électrique des zones actives 6a, 6b et 6c, lorsque ces zones actives ne sont pas en contact avec une substance qui soit électriquement conductrice en association avec l'une desdites zones actives, est très élevée, notamment quasi infinie.

Pour pouvoir contrôler si les connexions électriques entre le contact électrique 24 et la plage de contact 8 et entre le contact électrique 27 et la plage de contact 10 sont correctement établies, les contacts électriques 24 et 27 sont susceptibles d'être reliés électriquement à une source de tension (non-représentée) appartenant au moyen de mesure 30 et/ou à l'électronique de contrôle 32. Ensuite, il est prévu que la résistance de contrôle 34 ait une valeur permettant l'établissement d'un courant électrique de contrôle IC lorsque les contacts électriques 24 et 27 sont reliés à ladite source de tension. La résistance de contrôle 34 a par exemple une valeur de l'ordre de quelques dizaines de KΩ, en particulier 30 KΩ.

De manière avantageuse, la source de tension servant à établir un courant électrique de contrôle IC est une source de tension constante. De ce fait, connaissant la valeur de la résistance de contrôle 34, il est possible de déterminer la valeur minimale d'un courant électrique de contrôle IC devant circuler à travers la résistance de contrôle 34 lorsque la connexion électrique entre les quatre contacts électriques 24, 25, 26 et 27 et les deux plages de contact électrique 8 et 10 est correctement établie.

Afin de contrôler si ladite valeur minimale est atteinte lors d'une phase de contrôle, c'est-à-dire une phase dans laquelle les zones actives 6a, 6b et 6c ne sont en contact avec aucune substance qui soit électriquement conductrice en association avec l'une de ces zones actives, les moyens de contrôle 32 comprennent des moyens de comparaison (non représentés) permettant de comparer la valeur du courant électrique de contrôle IC à une première valeur de référence sensiblement égale à ladite valeur minimale.

Dans une variante de ce premier mode de réalisation, le courant de contrôle IC peut être enregistré, la valeur de ce courant de contrôle IC étant prise en considération par les moyens de mesure 30 lors d'une mesure d'une substance à analyser au moyen d'au moins une des zones actives 6a, 6b et 6c du capteur amovible associé au dispositif de mesure selon ce premier mode de réalisation de l'invention.

On notera aussi que la valeur de la résistance de contrôle 34, reliée électriquement en parallèle avec les zones actives 6a, 6b et 6c aux moyens de mesure 30, est à prendre en considération dans l'agencement des moyens de mesure 30. Pour que la sensibilité du dispositif de mesure agencé selon ce premier mode de réalisation soit grande, il est préférable que la résistance électrique de chacune des zones actives 6a, 6b et 6c, lorsqu'elle est mise en contact avec une substance à analyser, ait une valeur inférieure à la valeur de la résistance de contrôle 34, par exemple dix fois plus petite.

Dans une variante de ce premier mode de réalisaion, lesdits moyens de comparaison permettant également de comparer la valeur du courant de contrôle IC à une deuxième valeur de référence. Le capteur amovible, associé au dispositif de mesure selon cette variante, est considéré être défectueux lorsque la valeur du courant de contrôle IC est supérieure à cette deuxième valeur de référence. Cette comparaison permet de détecter notamment une défectuosité d'une des zones actives 6a, 6b ou 6c ou un court-circuit intervenant dans le capteur amovible.

Ainsi, lors d'une phase de contrôle préalable à une mesure, le dispositif de mesure associé à un capteur amovible est jugé en état de fonctionnement correct lorsque la valeur du courant de contrôle IC est comprise entre la première valeur de référence et la deuxième valeur de référence. A cet effet, les moyens de contrôle du dispositif de mesure selon l'invention sont agencés pour fournir une information de non-fonctionnement de ce dispositif de mesure associé à un capteur amovible introduit dans ce dernier lorsque la valeur du courant de contrôle est inférieure à ladite première valeur de référence ou supérieure à ladite deuxième valeur de référence.

En se référant à la figure 5, on décrira ci-après un deuxième mode de réalisation du circuit électrique d'un dispositif de mesure selon l'invention.

Sur cette figure 5 sont également représentées les deux plages de contact électrique 8 et 10 d'un capteur amovible et une zone active 6 de ce capteur amovible.

Chacune des deux plages de contact électrique 8 et 10 est également associée à deux contacts électriques 24 et 26, 25 et 27 appartenant au dispositif de mesure. Ce dispositif de mesure comprend en outre des moyens de mesure 40 et des moyens de contrôle comprenant une électronique de contrôle 42 et deux commutateurs 44 et 46. Chacun des deux commutateurs 44 et 46 peut être actionné indépendamment l'un de l'autre par l'électronique de contrôle 42. Les deux commutateurs 44 et 46 sont reliés électriquement au moyen de mesure 40, chacun de ces deux commutateurs 44 et 46 étant susceptible de commuter entre une première borne 48, 49 et une deuxième borne 50, 51.

Les deux bornes 48 et 50 du commutateur 44 sont reliées respectivement aux contacts électriques 24 et 25, lesquels sont respectivement associés aux deux plages de contact électrique 8 et 10 du capteur amovible. De même, les deux bornes 49 et 51 du commutateur 46 sont reliées respectivement aux deux contacts électriques 26 et 27, lesquels sont respectivement associés aux deux plages de contact électrique 8 et 10. Les moyens de mesure 40 et/ou l'électronique de contrôle 42 comprennent une source de tension (non représentée) susceptible d'être reliée en série aux deux commutateurs 44 et 46.

Ce second mode de réalisation de l'invention permet de contrôler séparément la connexion électrique des contacts électriques 24 et 26 associés à la plage de contact 8 et la connexion électrique des contacts électriques 25 et 27 associés à la plage de contact électrique 10.

Lorsque les commutateurs 44 et 46 sont reliés respectivement aux bornes 48 et 49, il est possible de contrôler si un courant électrique de contrôle circule entre les contacts électriques 24 et 26 lorsque les deux commutateurs 44 et 46 sont reliés en série à ladite source de tension. De même, lorsque les deux commutateurs 44 et 46 sont reliés respectivement aux bornes 50 et 51, il est possible de contrôler si la connexion électrique entre les contacts électriques 25 et 27 et la plage de contact électrique 10 est correctement établie.

Ainsi, il est possible de contrôler indépendamment l'état des connexions électriques de chacune des deux plages de contact électrique 8 et 10. De plus, ce deuxième mode de réalisation de l'invention permet de contrôler la connexion électrique entre le capteur amovible et le dispositif de mesure sans que la résistance au passage d'un courant électrique de la zone active 6 ou l'état dans lequel cette zone active 6 se trouve ait une influence sur le contrôle effectué.

A nouveau, l'électronique de contrôle 42 comporte des moyens de comparaison permettant de comparer la valeur d'un courant électrique de contrôle circulant soit entre les contacts électriques 24 et 26, soit entre les contacts électriques 25 et 27 à une première valeur de référence correspondant à un courant minimal au-dessous duquel la connexion électrique est considérée incorrectement établie.

Ce second mode de réalisation permet également de contrôler l'état de la zone active 6 en commutant le commutateur 44 sur la borne 48 et le commutateur 46 sur la borne 51 ou en commutant le commutateur 44 sur la borne 50 et le commutateur 46 sur la borne 49. Il est ainsi possible de contrôler si la résistance de la zone active 6 du capteur amovible au passage d'un courant électrique, lorsque cette zone active 6 n'est en contact avec aucune substance qui soit électriquement conductrice en association avec cette zone active, n'est pas inférieure à une valeur déterminée.

Pour effectuer ce dernier contrôle, les moyens de comparaison de l'électronique de contrôle 42 permettent de comparer la valeur d'un courant électrique de contrôle susceptible de circuler à travers la zone active 6 à une deuxième valeur de référence. Lorsque ce dernier courant électrique de contrôle a une valeur inférieure à cette deuxième valeur de référence, le capteur amovible est considéré être défectueux. Cette défectuosité peut venir par exemple de la zone active elle-même ou d'un court-circuit entre les deux conducteurs 54 et 56 reliant électriquement la zone active 6 aux plages de contact 8 et 10.

Finalement, on notera que le second mode de réalisation de l'invention est également adapté à des capteurs amovibles multizones, tout comme le premier mode de réalisation décrit à la figure 4 est également adapté à des capteurs amovibles monozones.

## Revendications

1. Dispositif de mesure (20) destiné à être associé à un capteur amovible (4), présentant au moins deux plages de contact électrique (8,10),et comprenant des moyens de mesure (30;40) et des moyens de connexion (22) comportant au moins deux premiers moyens de contact (24,27) reliés électriquement à une source de tension, ces deux premiers moyens de contact (24, 27) servant respectivement à établir une connexion électrique entre lesdites deux plages de contact dudit capteur amovible et ces moyens de mesure, ce dispositif de mesure étant caractérisé en ce que lesdits moyens de connexion (22) comportent en outre au moins deux deuxièmes moyens de contact (25,26), ces deux deuxièmes moyens de contact servant respectivement à relier électriquement lesdites deux plages de contact dudit capteur amovible à des moyens de contrôle (32,34;42,44,46) permettant de contrôler ladite connexion électrique entre chacune desdites deux premières plages de contact et lesdits moyens de mesure.

2. Dispositif selon la revendication 1, lesdits moyens de mesure (30;40) permettant de mesurer un courant électrique (IC) susceptible de circuler entre ces deux premiers moyens de contact, ce dispositif de mesure étant caractérisé en ce que lesdits moyens de contrôle (32,34) comprennent une résistance électrique de contrôle (34) reliée électriquement en série avec les deux deuxièmes moyens de contact (25,26) et des moyens de comparaison permettant de comparer la valeur dudit courant électrique (IC) à une première valeur de référence, lesdits moyens de contrôle étant agencés pour fournir une information de non-fonctionnement dudit dispositif de mesure associé audit capteur amovible lorsque ladite valeur dudit courant électrique est inférieure à ladite première valeur de référence.

3. Dispositif de mesure selon la revendication 2, ledit capteur amovible (4) comprenant au moins une zone active (6;6a,6b,6c) destinée à être mise en contact avec une substance à analyser et reliée électriquement en série avec les deux plages de contact (8,10), cette zone active ayant une résistance élevée au passage d'un courant électrique entre les deux plages de contact lorsque cette zone active n'est en contact avec aucune substance qui soit électriquement conductrice en association avec ladite zone active, ce dispositif de mesure étant caractérisé en ce que lesdits moyens de comparaison permettent de comparer la valeur dudit courant électrique (IC) à une deuxième valeur de référence, lesdits moyens de contrôle étant agencés pour fournir une information de non-fonctionnement dudit dispositif de mesure associé audit capteur amovible lorsque ladite valeur dudit courant électrique est supérieure à ladite deuxième valeur de référence.

4. Dispositif de mesure selon la revendication 1, caractérisé en ce que lesdits moyens de contrôle (42,44,46) comprennent un premier commutateur (44), susceptible de commuter entre une première borne (48) et une deuxième borne (50), et un deuxième commutateur (46) susceptible de commuter entre une troisième borne (51) et une quatrième borne (49), lesdites première et troisième bornes (48,51) étant reliées respectivement aux deux premiers moyens de contact (24,27), lesdites deuxième et quatrième bornes (50,49) étant reliées respectivement aux deux deuxièmes moyens de contact (25,26), lesdits moyens de mesure comprenant une source de tension susceptible d'être reliée électriquement en série auxdits premier et deuxième commutateurs (44,46) et permettant de mesurer un courant électrique susceptible de circuler entre ces premier et deuxième commutateurs, lesdits moyens de contrôle permettant d'actionner indépendamment lesdits premier et deuxième commutateurs et comprenant des moyens de comparaison permettant de comparer la valeur dudit courant électrique à une première valeur de référence, ces moyens de contrôle étant agencés pour fournir une information de non-fonctionnement dudit dispositif de mesure associé audit capteur amovible lorsque ladite valeur dudit courant électrique est inférieur à ladite première valeur de référence.

5. Dispositif de mesure selon la revendication 4, ledit capteur amovible comprenant au moins une zone active (6;6a,6b,6c) destinée à être mise en contact avec une substance à analyser et reliée électriquement en série avec les deux plages de contact (8,10), ladite zone active ayant une résistance élevée au passage d'un courant électrique entre les deux plages de contact lorsque cette zone active n'est en contact avec aucune substance qui soit électriquement conductrice en association avec cette zone active, ce dispositif de mesure étant caractérisé en ce que lesdits moyens de comparaison permettent de comparer la valeur dudit courant électrique à une deuxième valeur de référence, lesdits moyens de contrôle étant agencés pour fournir une information de non-fonctionnement dudit dispositif de mesure associé audit capteur amovible lorsque ladite valeur dudit courant électrique est supérieure à ladite deuxième valeur de référence.

6. Dispositif de mesure selon l'une quelconque des revendications précédentes, caractérisé en ce que ce dispositif de mesure et ledit capteur amovible qui lui est associé sont agencés pour mesurer le taux de glucose dans le sang.

## Claims

1. Measuring device (20) intended to be used with a removable sensor (4), having at least two electrical contact surfaces (8,10), and comprising measuring means (30;40) and connecting means (22) comprising at least two first contact means (24,27) electrically connected to a voltage source, these two first contact means (24,27) being used respectively to establish an electrical connection between said two contact surfaces of said removable sensor and these measuring means, this measuring device being characterized in that said connecting means (22) also comprises at least two second contact means (25,26), these two second contact means being used respectively to connect electrically said two contact surfaces of said removable sensor to testing means (32,34; 42,44,46) enabling said electrical connection between each of said first contact surfaces and said measuring means to be tested.

2. Measuring device according to claim 1, said measuring means (30;40) enabling an electric current (IC) capable of flowing between these two first contact means to be measured, this measuring device being characterized in that said testing means (32,34) comprises an electrical testing resistor (34) electrically connected in series with said two second contact means (25,26) and comparing means enabling the value of said electric current (IC) to be compared to a first reference value, said testing means being arranged to indicate that said measuring device used with said removable sensor is not functioning when said value of said electric current is less than said first reference value.

3. Measuring device according to claim 2, said removable sensor (4) comprising at least one active zone (6;6a,6b,6c) intended to be placed in contact with a substance to be analysed and electrically connected in series with the two contact surfaces (8, 10), this active zone having a high resistance to the flow of an electric current between the two contact surfaces when this active zone is not in contact with any substance which is electrically conductive in conjunction with said active zone, this measuring device being characterized in that said comparing means enables the value of said electric current (IC) to be compared to a second reference value, said testing means being arranged to indicate that said measuring device used with said removable sensor is not functioning when said value of said electric current is greater than said second reference value.

4. Measuring device according to claim 1, characterized in that said testing means (42,44,46) comprises a first switch (44) capable of switching between a first terminal (48) and a second terminal (50) and a second switch (46) capable of switching between a third terminal (51) and a fourth terminal (49), said first and third terminals (48,51) being connected respectively to said two first contact means (24,27), said second and fourth terminals (50,49) being connected respectively to said two second contact means (25,26), said measuring means comprising a voltage supply intended to be electrically connected in series to said first and second switches (44,46) and enabling an electric current capable of flowing between these first and second switches to be measured, said testing means enabling said first and second switches to be activated independently and comprising comparing means enabling the value of said electric current to be compared to a first reference value, this testing means being arranged to indicate that said measuring device used with said removable sensor is not functioning when said value of said electric current is less than said first reference value.

5. Measuring device according to claim 4, said removable sensor comprising at least one active zone (6;6a,6b,6c) intended to be placed in contact with a substance to be analysed and electrically connected in series to said two contact surfaces (8,10), said active zone having a high resistance to the flow of an electric current between the two contact surfaces when this active zone is not in contact with any substance which is electrically conductive in conjunction with this active zone, this measuring device being characterized in that said comparing means enables the value of said electric current to be compared to a second reference value, said testing means being arranged to indicate that said measuring device used with said removable sensor is not functioning when said value of said electric current is greater than said second reference value.

6. Measuring device according to any one of the preceding claims, characterized in that this measuring device and said removable sensor with which it is used are arranged to measure glucose levels in blood.

## Patentansprüche

1. Meßvorrichtung (20), die dazu bestimmt ist, mit einem entnehmbaren Sensor (4) verbunden zu werden, der wenigstens zwei elektrische Kontaktbereiche (8, 10) aufweist und Meßmittel (30; 40) und Anschlußmittel (22)umfaßt, die wenigstens zwei erste Kontaktmittel (24, 27) umfassen, die mit einer Spannungsquelle elektrisch verbunden sind und dazu dienen, eine elektrische Verbindung zwischen den beiden Kontaktbereichen des entnehmbaren Sensors und den Meßmitteln herzustellen, wobei die Meßvorrichtung dadurch gekennzeichnet ist, daß die Verbindungsmittel (22) außerdem wenigstens zwei zweite Kontaktmittel (25, 26) umfassen, die dazu dienen, die zwei Kontaktbereiche des entnehmbaren Sensors mit Steuermitteln (32, 34; 42, 44, 46) elektrisch zu verbinden, die es ermöglichen, die elektrische Verbindung zwischen jedem der zwei ersten Kontaktbereiche und den Meßmitteln zu steuern.

2. Vorrichtung nach Anspruch 1, wobei es die Meßmittel (30; 40) ermöglichen, einen elektrischen Strom (IC) zu messen, der zwischen den beiden ersten Kontaktmitteln fließen kann, wobei die Meßvorrichtung dadurch gekennzeichnet ist, daß die Steuermittel (32, 34) einen elektrischen Steuerwiderstand (34), der mit den beiden ersten Kontaktmitteln (25, 26) elektrisch in Reihe geschaltet ist, sowie Vergleichsmittel umfassen, die es ermöglichen, den Wert des elektrischen Stroms (IC) mit einem ersten Referenzwert zu vergleichen, wobei die Steuermittel so beschaffen sind, daß sie eine Information bezüglich einer Betriebsunterbrechung der dem entnehmbaren Sensor zugeordneten Meßvorrichtung liefern, wenn der Wert des elektrischen Stroms kleiner als der erste Referenzwert ist.

3. Meßvorrichtung nach Anspruch 2, wobei der entnehmbare Sensor (4) wenigstens eine aktive Zone (6; 6a, 6b, 6c) aufweist, die dazu bestimmt ist, mit einer zu analysierenden Substanz in Kontakt gebracht zu werden, und mit den beiden Kontaktbereichen (8, 10) elektrisch in Reihe geschaltet ist, wobei diese aktive Zone für den Durchgang eines elektrischen Stroms zwischen den beiden Kontaktbereichen einen hohen Widerstand besitzt, wenn diese aktive Zone mit keinerlei Substanz in Kontakt ist, die in Verbindung mit der aktiven Zone elektrisch leitend ist, wobei die Meßvorrichtung dadurch gekennzeichnet ist, daß die Vergleichsmittel ermöglichen, den Wert des elektrischen Stroms (IC) mit einem zweiten Referenzwert zu vergleichen, wobei die Steuermittel so beschaffen sind, daß sie eine Information bezüglich einer Betriebsunterbrechung der dem entnehmbaren Sensor zugeordneten Meßvorrichtung liefern, wenn der Wert des elektrischen Stroms größer als der zweite Referenzwert ist.

4. Meßvorrichtung nach Anspruch 1, dadurch gekennzeichnet, daß die Steuermittel (42, 44, 46) einen ersten Umschalter (44), der zwischen einem ersten Anschluß (48) und einem zweiten Anschluß (50) umschalten kann, und einen zweiten Umschalter (46), der zwischen einem dritten Anschluß (51) und einem vierten Anschluß (49) umschalten kann, umfassen, wobei der erste und der dritte Anschluß (48, 51) jeweils mit einem der beiden ersten Kontaktmittel (24, 27) verbunden sind, während der zweite und der vierte Anschluß (50, 49) jeweils mit einem der beiden zweiten Kontaktmittel (25, 26) verbunden sind, wobei die Meßmittel eine Spannungsquelle aufweisen, die mit den ersten und zweiten Umschaltern (44, 46) elektrisch in Reihe geschaltet werden können, und es ermöglichen, einen elektrischen Strom zu messen, der zwischen diesen ersten und zweiten Umschaltern fließen kann, wobei die Steuermittel es ermöglichen, die ersten und zweiten Umschalter unabhängig zu betätigen, und Vergleichsmittel aufweisen, die es ermöglichen, den Wert des elektrischen Stroms mit einem ersten Referenzwert zu vergleichen, wobei diese Steuermittel so beschaffen sind, daß sie eine Information bezüglich einer Betriebsunterbrechung der dem entnehmbaren Sensor zugeordneten Meßvorrichtung liefern, wenn der Wert des elektrischen Stroms kleiner als der erste Referenzwert ist.

5. Meßvorrichtung nach Anspruch 4, wobei der entnehmbare Sensor wenigstens eine aktive Zone (6; 6a, 6b, 6c) aufweist, die dazu vorgesehen ist, mit einer zu analysierenden Substanz in Kontakt zu gelangen, und mit den beiden Kontaktbereichen (10) elektrisch in Reihe geschaltet zu werden, wobei die aktive Zone für den Durchgang eines elektrischen Stroms zwischen den beiden Kontaktbereichen einen hohen Widerstand besitzt, wenn diese aktive Zone mit keinerlei Substanz in Kontakt ist, die in Verbindung mit dieser aktiven Zone elektrisch leitend ist, wobei die Meßvorrichtung dadurch gekennzeichnet ist, daß die Vergleichsmittel es ermöglichen, den Wert des elektrischen Stroms mit einem zweiten Referenzwert zu vergleichen, wobei die Steuermittel so beschaffen sind, daß sie eine Information bezüglich einer Betriebsunterbrechung der dem entnehmbaren Sensor zugeordneten Meßvorrichtung liefern, wenn der Wert des elektrischen Stroms größer als der zweite Referenzwert ist.

6. Meßvorrichtung nach einem der vorhergehenden Ansprüche, dadurch gekennzeichnet, daß die Meßvorrichtung und der ihr zugeordnete entnehmbare Sensor so beschaffen sind, daß sie den Glukoseanteil im Blut messen.
